# EUROPÄISCHE PATENTSCHRIFT

(11) **EP 1 731 197 B1**
(45) Veröffentlichungstag und Bekanntmachung des Hinweises auf die Patenterteilung: **10.12.2008**
(21) Anmeldenummer: 06010794.3
(22) Anmeldetag: 26.05.2006
(51) Int. Cl.: A61Q 19/00, A61K 8/88, A61K 8/73

(54) **Topische kosmetische Formulierungen zur Regulierung und Verbesserung des Feuchtigkeitsgehalts der Haut**
Topical cosmetic formulations for regulating and improving the moisture content in skin
Formulations cosmétiques topiques pour la régulation et l'augmentation de la teneur en humidité de la peau

(30) Priorität: 07.06.2005 DE 102005026069
(43) Veröffentlichungstag der Anmeldung: 13.12.2006
(73) Patentinhaber: Evonik Goldschmidt GmbH, 45127 Essen (DE)
(72) Erfinder: Blasko-Begoihn, Heike, 45359 Essen (DE); Farwick, Mike, Dr., 45138 Essen (DE); Lersch, Peter, Dr., 46537 Dinslaken (DE); Maczkiewitz, Ursula, 45219 Essen (DE); Mecking, Maria, 46244 Bottrop (DE); Scheuermann, Ralph, Dr., 45359 Essen (DE); Weitemeyer, Christian, Dr., 45259 Essen (DE)

(56) Entgegenhaltungen:
- EP-A- 0 774 247
- EP-A- 0 891 768
- EP-A- 0 958 811
- WO-A-02/19981
- DE-A1- 19 710 369
- US-A1- 2003 165 546
- US-B1- 6 723 688
- PATENT ABSTRACTS OF JAPAN Bd. 2003, Nr. 12, 5. Dezember 2003 (2003-12-05) -& JP 2005 120032 A (NIPPON FINE CHEM CO LTD), 12. Mai 2005 (2005-05-12)
- PATENT ABSTRACTS OF JAPAN Bd. 1998, Nr. 10, 31. August 1998 (1998-08-31) -& JP 10 139889 A (JSR CORP), 26. Mai 1998 (1998-05-26)

## Beschreibung

Gegenstand der Erfindung sind neue kosmetische Formulierungen zur lokalen topischen Behandlung von Körperteilen die einen Gehalt an Hautfeuchtigkeit steigernden Substanzen aufweisen, die zum Erhalten und zur Behandlung der epidermalen Hornschicht vorgesehen sind, insbesondere unter gleichzeitiger Gewährleistung einer Hydratation, einer dauerhaften Verbesserung des Wasserhaushalts und einer Verringerung der Rauheit der Haut.

Die Hornhaut, welche die äußerste Schicht der Haut darstellt, ist als wichtige Barriereschicht von besonderer Bedeutung für den Schutz vor Umwelteinflüssen. Zur Erhaltung ihrer Glätte, Elastizität und Geschmeidigkeit benötigt die Haut neben Lipiden auch ein Optimum an Wasser. Diese Erkenntnisse wurden in grundlegenden Arbeiten u. a. von Jacobi sowie Schuleit und Szakall bestätigt. Der Mensch gibt täglich mehrere Deziliter bis zu mehreren Litern Wasser über die Haut an die Umwelt ab. Das in der Haut befindliche Wasser stammt aus verschiedenen Quellen und liegt nach neueren Erkenntnissen sowohl als Dampf als auch in flüssiger Form sowie adsorbiert an Proteinen vor. Es ist nicht bekannt, wieviel Wasser die Epidermis enthält. Da einige Schichten in der Hornschicht bis zu 70 % an Proteinen und Lipiden enthalten, kann man von bis zu 30 % Wasser ausgehen. Als sicher angenommen werden kann, dass Wasser durch verschiedene Hautschichten wandern kann.

Für die Diffusion des Wassers durch die Hautschichten gibt es dabei verschiedene Modelle, von denen bis jetzt noch keines schlüssig bewiesen werden konnte.

Analog zu hydrophoben Substanzen, welche durch Lipidporen in die Hornschicht (Stratum Corneum, SC) penetrieren können, soll das Wasser durch spezifische Wasserporen "aqueous pores" transportiert werden. Diese Poren sollen einen Durchmesser von 15 bis 25 Å besitzen.

Ein anderer Ansatz postuliert, dass das Stratum Corneum von wassergefüllten Kanälen durchzogen sein soll. Durch Beugungsexperimente mit Röntgenstrahlen konnte gezeigt werden, dass in einem Lipid-Doppelschichtsystem Lücken bestehen, die groß genug sind, dass sich dort kondensiertes Wasser sammeln kann.

Zur Feuchtigkeitsregulation der Haut ist also unzweifelhaft neben einer intakten Permeabilitätsbarriere auch das Vorhandensein von wasserbindenden Substanzen erforderlich, die in den epidermalen Hornschichten gebildet werden. Die in der Epidermis enthaltenen natürlichen Feuchthaltestoffe NMF (natural moisturizing factors) binden Feuchtigkeit in der Haut. Sie stellen ein Gemisch verschiedener Verbindungen dar und bestehen aus 40 % Aminosäuren, 12 % Pyrrolidoncarbonsäure, 7 % Harnstoff sowie 41 % anorganischen und organischen Salzen (hauptsächlich Lactate).

Ein erhöhter transepidermaler Wasserverlust (TEWL) bewirkt eine Austrocknung der Haut. Es wird angenommen, dass der Verlust der natürlichen Feuchthaltestoffe mit einer Verringerung des Wassergehalts und einer reduzierten Weichheit der Keratinschicht korreliert.

Drastische Umweltbedingungen, wie niedrige Temperaturen oder zu geringe Feuchtigkeit im Winter, tragen in erheblichem Maße mit dazu bei, dass die Haut rau und trocken wird. Die in der Epidermis enthaltenden Feuchthaltestoffe werden ebenso leicht durch häufiges Waschen oder Baden insbesondere bei gleichzeitiger Einwirkung von Wasch- oder Reinigungsmitteln herausgelöst. So kann mehr Wasser aus tiefer gelegenen Hautschichten entweichen und der transepidermale Wasserverlust nimmt zu.
Sensorisch manifestiert sich dies durch Symptome wie z. B. eine vermehrt raue, schuppige, glanzlose und stumpf wirkende Hautoberfläche. Ein Flexibilitätsverlust und eine Beeinträchtigung der Barrierefunktion der Haut, die von der Wasserbindungskapazität der Hornschicht abhängt, sind die Folge. Dadurch wird der Wassergehalt der Hornschicht weiter reduziert.

Die Befeuchtung und Feuchtigkeitsregulierung der Haut sowie eine sorgfältige Pflege zur Verhinderung einer andauernd trockenen Haut sind nicht nur ein ästhetisches Bedürfnis, sondern auch ein probates Mittel, um chronischen Hautkrankheiten effektiv vorzubeugen.

Eine Vielzahl von in-vivo Methoden, mit denen der Feuchtigkeitsgehalt der Haut ermittelt werden kann, sind bekannt. Dabei werden besonders Leitfähigkeit und die dielektrischen Eigenschaften (Kapazität) der Hornschicht zur Bestimmung der corneometrischen Hautfeuchtigkeit eingesetzt. Zur Verfügung stehende Meßgeräte mit Hilfe derer Wassergehalt des Stratum Corneums ermittelt werden kann, beinhalten die Corneometer-Typen CM 820 und CM 825 (Courage + Khazaka) sowie das "dermal phase meter" Skicon 200 (Nova). Diese nicht invasiven und einfachen Methoden erlauben es, die Veränderung der Hornschichthydratation quantitativ zu messen.

Die Viscoelastizität der Haut kann über das Dermal Torque Meter (DiaStron) oder auch über das Cutometer (Courage + Khazaka) ermittelt werden.

Um einem trockenen Hautzustand entgegenzuwirken und den Wasserhaushalt der Haut wiederherzustellen gibt es eine Reihe von kosmetischen Zubereitungen mit hydroregulativer Wirkung. Diese Präparate sind in Form von Emulsionen ideale Zubereitungen, um der Haut Fett und Feuchtigkeit zuzuführen und enthalten in der Regel eine Reihe von Wirkstoffen, die beim Auftragen eine schützende Funktion entfalten, dadurch den Zustand der Hautoberfläche verbessern und den funktionellen Zustand der Haut verändern, indem sie z. B. regulierend auf die Hautfeuchte einwirken und durch das Eindringen unter die Hautoberfläche pflegende Eigenschaften zur Wirkung kommen. Es existieren verschiedene Mechanismen wie der Wassergehalt der Epidermis durch kosmetische Inhaltsstoffe und Formulierungen positiv beeinflusst werden kann.

Das Verdampfen von Wasser aus den oberen Hautschichten kann durch einen okklusiven Lipid- oder Polymerfilm unterbunden werden. Dadurch wird von den unteren Hautschichten Wasser an die oberen abgegeben sowie die Schweißbildung vermindert, wodurch die Hautfeuchtigkeit der oberen Schichten des SC stark ansteigt. Unter solchen okklusiven Bedingungen kommt es aber typischerweise zu einem Wasserstau in der Haut und einer vermehrten endogenen Quellung der Hornschicht, wodurch die Regenerationsfähigkeit der Haut verlangsamt wird.

Formulierungstechnisch ist es möglich, kosmetische Produkte herzustellen, die mehr Wasser enthalten als die äußeren Hautschichten und somit Wasser an das Sratum Corneum abgeben. Spezielle Lipide sind ebenfalls in der Lage den transepidermalen Wasserverlust zu reduzieren und können daher auch als eine Art Moisturizer angesehen werden.

Ein weiterer gebräuchlicher Ansatz ist der Zusatz von Feuchthaltemitteln als aktivierende Inhaltsstoffe zu kosmetischen Emulsionen, welche die Versorgung der Keratinschicht mit einer ausreichenden Menge Feuchtigkeit über definierte zeitabschnitte sicherstellen sollen. Feuchthaltemittel werden auch als Moisturizer oder Humectants bezeichnet und sollen einerseits Wasser in der Epidermis zurückhalten andererseits durch Stabilisierung der Barrierefunktion in der oberen Hornschicht den TEWL vermindern.

Eine Vielzahl solcher Substanzen ist beschrieben und wird bereits verwendet. Diese sind in der Regel hygroskopische Substanzen mit der Fähigkeit, Wasser mehr oder weniger stark zu binden und die ausgewaschenen natürlichen Stoffe ganz oder teilweise zu ersetzen. Prinzipiell gehören dazu hygroskopische Substanzen wie mehrwertige Alkohole, ethoxylierte Polyole, Zucker sowie Polysaccharide, Aminosäuren und Proteinhydrolysate.

Zu den wichtigsten Vertretern dieser Feuchthaltemittel zählt Harnstoff, der ein effektives Feuchthaltemittel mit guter Wasserbindungskapazität ist. Harnstoff kann zudem die Permeation zusätzlicher Wirkstoffe in die Haut fördern. Auch die essentielle Aminosäure Arginin zählt neben dem Harnstoff zu natürlichen Feuchthaltefaktoren. Bekannt ist weiterhin ein Wirksystem aus Arginin, Harnstoff und Glycerol, welches eine hydratisierende Wirkung in der Hornschicht bewirkt und gegen Hauttrockenheit hilft und vorbeugt.

Ein weiterer wichtiger Vertreter ist Glyzerin (Glycerol, 1,2,3 Propantriol). Es wird angenommen, dass Glycerin an oder nahe der Hautoberfläche bleibt. Bei sehr niedriger Luftfeuchtigkeit der Umgebung wird Wasser durch die Haut nach außen abgegeben. Das Glycerin ersetzt diesen Wasserverlust, indem es Wasser aus den unteren Hautschichten absorbiert, d. h. es regt die Zirkulation von Wasser durch die Epidermis an. Neuere Publikationen erklären den Moistrizer-Effekt von Glycerin mit seiner Fähigkeit, bestimmte Enzym-Reaktionen, die für eine Wiederherstellung eines intakten SC erforderlich sind, zu fördern.

Ebenfalls sehr effektives Feuchthaltemittel sind die Hyaluronsäuren bzw. deren Salze. Hyaluronsäure ist ein polymeres Glycosaminoglucan, welches mit anderen Gewebekomponenten assoziert vorliegt und wichtig zur Kontrolle der Gewebehydration ist. Hyaluronsäure verfügt über eine enorme Wasserbindungsfähigkeit und hält das Wasser im Stratum corneum, wodurch die Elastizität und Weichheit der Haut gesteigert wird. Ein abnehmender Anteil an Hyaluronsäure in der Haut korreliert mit Hautalterung und Faltenbildung.

An Versuchen die Hautfeuchtigkeit durch Zusatz von Inhaltsstoffen positiv zu beeinflussen hat es bisher also nicht gemangelt und es wurden in der Vergangenheit bereits eine Vielzahl unterschiedlicher Substanzen vorgeschlagen, welche ihrerseits in verschiedene Regulationsmechanismen der Hautfeuchte-Regulation eingreifen. So werden in der Patentliteratur eine Reihe von feuchtigkeitsbindenden Substanzen und ausgewogene Abmischungen beschrieben, welche eine regulative Wirkung auf die Hautfeuchte haben.

Beispiele für den Einsatz von Aminosäuren als Feuchthaltemittel finden sich in den Patentschriften US 4 772 591, US 3 849 576, US 5 135 913, US 4 859 653 und US 4 760 051 sowie in der europäischen Patentschrift EP-B 0 070 048. Diese beschreiben spezifische Vitamine und Aminosäuren entweder alleine oder in einer Vielzahl von Kombinationen. Darin sind auch bestimmte Formulierungen eingeschlossen, die bestimmte Aminosäurekomplexe beschreiben, die von den Aminosäuren wie Prolin, Arginin, Pyrrolidoncarbonsäure oder Glycin gebildet werden und dazu dienen sollen, in die Haut zu penetrieren, um die Hautfeuchtigkeit zurückzuhalten und die Homeostase der Epidermis zu fördern und das Zellwachstum anzuregen.

Aus der WO-A-00/15187 (SKW Trostberg) ist die Verwendung von Kreatin als Feuchthaltemittel in kosmetischen Zubereitungen bekannt. Der Einsatz von Kreatin bzw. dessen geeignete Derivate soll Symptome einer trockenen Haut wie Risse und Schuppung nachhaltig beseitigen.

Im US-Patent 5 254 331 wird eine Hautcreme beschrieben, welche einen Protein-Aminosäure-Vitamin-Nukleotid-Komplex enthält. Dieser Komplex besteht aus Propylenglykol, Serumproteinen, Niacinamid, Wasser, Adenosinphosphat und Arginin. Diese Bestandteile sollen die Regeneration der epidermalen Zellen anregen und die Hautfeuchte andauernd und nachhaltig verbessern.

In der US 5 215 759 werden kosmetische wässrige Formulierungen beschrieben, welche hautbefeuchtende Mischungen aus 20 bis 40 % Glycerin, 10 bis 20 % Chitin, 5 bis 15 % Natriumlactat, 1 bis 5 % Natriumchlorid, 5 bis 15 % Natriumpyrrolidoncarboxylat, jeweils 1 bis 5 % Glycogen, Harnstoff und Propylenglykol sowie mindestens 1 % jeweils einer Aminosäure ausgewählt aus einer Gruppe der Aminosäuren, die Glycin, Arginin, Lysin, Histidin und Ornithin oder Placenta-Protein umfasst, als Bestandteile enthalten.

Vorzugsweise soll eine solche Substanz bereits in geringen Einsatzkonzentrationen eine deutliche Wirkung hervorrufen, nicht toxisch, vorzugsweise natürlichen Ursprungs sein, sehr gut von der Haut toleriert werden, eine hohe Verträglichkeit mit anderen Inhaltsstoffen aufweisen, eine gute Langzeitstabilität aufweisen und sich problemlos in Hautbehandlungsmittel einarbeiten lassen.

Besonders wünschenswert ist es, wenn diese Substanzen zusätzlich auch einfach und kostengünstig hergestellt werden können und in einer Form anfallen, welche einfach aufgereinigt werden kann und damit die an kosmetische bzw. dermatologische Wirkstoffe gestellten hohen Reinheitsbedingungen erfüllt. Zusätzlich sollen die verwendeten Substanzen multifunktionelle Eigenschaften aufweisen, also neben der Normalisierung des Wassergehaltes der Haut weiterhin auch schützende, beruhigende und radikalfangende Eigenschaften aufweisen.

Trotz vieler Jahre Forschung auf dem Gebiet der Hautfeuchthaltemittel findet die Mehrheit der Anwender, dass die derzeitigen als Feuchthaltemittel verwendeten Substanzen bei genauer Betrachtung den an sie gestellten Ansprüchen jedoch nicht völlig gerecht werden.

Der Erfindung lag die Aufgabe zugrunde, eine solche nicht mit den geschilderten Nachteilen behaftete Wirkstoffkombination herzustellen, welche eine verbesserte Haut- und Schleimhautverträglichkeit aufweist und fähig ist, die Wasserbindungskapazität in der Epidermis und an der Hautoberfläche anzureichern, deren Wirkung über einen längeren Zeitraum hinweg gezielt einstellbar ist und gleichzeitig die Haut mit einem schützenden Film aus bioabbaubaren Stoffen bedeckt, deren Metabolite die Haut mit Nährstoffen versorgen und insgesamt die Haut effektiv in ihrem Restrukturierungsprozess unterstützen.

Ein Gegenstand der Erfindung sind somit kosmetische und/oder dermatologische Formulierungen zur topischen Behandlung der Haut zur Regulierung und Verbesserung des Feuchtigkeitsgehalts der Haut, enthaltend als Wirkstoffkombination
a) mindestens einer anionischen Polyaminosäure-Matrix und
b) mindestens einem Polysaccharid und gegebenenfalls
c) ein oder mehrere Osmoprotectanten
für die topische Anwendung, dadurch gekennzeichnet, dass die anionische Polyaminosäure eine Polyglutaminsäure mit einem mittleren Molgewicht von 1000000 bis 2000000 g/mol ist, und dass das Polysaccharid Scleroglucan mit einem mittleren Molgewicht von weniger als 600000 g/mol ist..

Ein weiterer Gegenstand der Erfindung sind kosmetische und/oder dermatologische Formulierungen zur topischen Behandlung der Haut, die gleichzeitig ein interpenetrierendes Netzwerk zur kontrollierten, verlangsamten Freisetzung der eingelagerten Wirkstoffe ausbilden.

Ein weiterer Gegenstand der Erfindung sind kosmetische und/oder dermatologische Formulierungen zur topischen Behandlung der Haut, die bezüglich der Wasserbindung ein Hystereseverhalten zeigen, d. h. es kommt zu einer verlangsamten Freisetzung des gebunden Wassers, sowohl auf der Hautoberfläche, als auch auf der Epidermis.

Weitere Gegenstände der Erfindung sind durch die Ansprüche gekennzeichnet.

Überraschenderweise wurde gefunden, dass eine Kombination einer anionischen Polyaminosäure-Matrix mit einer Polysaccharid-Matrix gemäß Anspruch 1 ein interpenetrierendes Polymernetzwerk ausbildet. Die Wahl der Komponenten für das interpenetrierende Polymernetzwerk stellt sicher, dass eine hohe Bioverträglichkeit gegeben ist und das resultierende hydrophile Feuchthaltemittel für kosmetische Zubereitungen zudem mit vielen anderen Roh- und Hilfsstoffen kompatibel ist. Weiterhin ist das Polymernetzwerk überraschenderweise fähig andere Hilfsstoffe einzulagern und nach der Applikation auf der Haut kontrolliert freizusetzen. Im Sinne der vorliegenden Erfindung hat es sich als besonders vorteilhaft erwiesen, wenn es sich bei den eingelagerten Hilfsstoffen um Osmoprotectanten wie z. B. Trimethylglycin, Arginin, Kreatin, Kaliumlactat, Taurin, Trehalose oder Myoinositol handelt, die in Mengen von 0,01 bis zu 40 Gew.-%, vorzugsweise bis 25 Gew.-% bezogen auf Gesamtformulierung, mitverwendet werden können. Das interpenetrierende Polymernetzwerk kann in den Mischungs-verhältnissen 1 : 99 bis 99 : 1 (bezogen auf die anionische Polyaminosäurematrix und das Polysaccharid) vorliegen, bevorzugt im Verhältnisrahmen 90 : 10 bis 10 : 90 und besonders bevorzugt im Verhältnisrahmen 80 : 20 bis 20 : 80.

Die auf diese Weise erhaltenen Mischungen können je nach Zusammensetzung in Bezug auf ihre Effektivität variabel eingestellt werden und gestatten die Anpassung an verschiedene Hauttypen.

Osmoprotectanten, auch kompatible Solute genannt, sind Substanzen, die die Haut vor schädigenden Umwelteinflüssen wie Trockenheit, Kälte, Hitze oder Salz schützen. Diese Substanzen ermöglichen ein Überleben unter widrigsten Bedingungen. Sie sind in hohem Maße mit dem menschlichen Zellstoffwechsel kompatibel und gut verträglich, so dass sie als kosmetischer Wirkstoff eingesetzt werden können.

Praktisch alle Materialien interagieren mehr oder weniger stark mit Feuchtigkeit. Die Kenntnis von feuchteinduzierten Änderungen der Materialeigenschaften ist ein wichtiger Schlüsselparameter für Entscheidungen hinsichtlich der Einsatzfähigkeit eines Produktes als Mittel zur Erhöhung der Hautfeuchte. Die Darstellung der Änderung des Wassergehalts einer Probe in Abhängigkeit von der relativen Luftfeuchtigkeit bei konstanter Temperatur wird als Wasserdampf Sorptionisotherme bezeichnet. Sie ist der einfachste und zugleich aussagekräftigste Weg, die Wechselwirkungen eines Materials mit Feuchtigkeit zu dokumentieren.

Aus der Quantität und dem Verlauf der Isothermen lassen sich wichtige Materialeigenschaften und Informationen hinsichlich ihrer Handhabung ableiten. Die Vorgänge bei der Sorption und Desorption sind meist sehr komplexe physikalisch-chemische Prozesse, weshalb die Isothermen nur experimentell bestimmt und nicht vorhergesagt werden können. Sorptionsisothermen sind die aussagekräftigsten thermodynamischen Einzelparameter für die Wechselwirkungen von Stoffen mit Wasser. Form und Verlauf der Isotherme geben Aufschluss über die Veränderungen im Material. Als Hysterese wird das Fortdauern einer Wirkung nach Wegfall der Ursache bezeichnet. Sie bezeichnet eine Erscheinung, die durch eine Hysteresekurve gekennzeichnet wird, typisch für die Hysterese ist das Auftreten von bistabilem Verhalten. Dies bedeutet, dass bei gleichen Umgebungsbedingungen der Zustand einer Substanz von der Vergangenheit abhängig ist.

Bezogen auf die Wasserbindungskapazität bei kosmetischen Wirkstoffen bedeutet eine Hysterese, dass Wasser bei Sorptions-Desorptionsprozessen verzögert abgegeben wird und somit die Hautfeuchtigkeit dauerhaft erhöht wird.

Im Sinne der vorliegenden Erfindung hat es sich als vorteilhaft erwiesen, wenn mindestens eine Polymerkomponente oder eine osmotische Schutzsubstanz ein Hystereseverhalten bei Sorptions-Desorptionsprozessen aufzeigt. Als besonders Vorteilhaft hat es sich herausgestellt, wenn sowohl mindestens eine Polymerkomponente als auch mindestens eine osmotische Schutzsubstanz ein Hystereseverhalten bei Sorptions-Desorptionsprozessen aufzeigt. Ein Gegenstand der Erfindung sind somit Formulierungen zur topischen Behandlung der Haut, die bezüglich der Wasserbindung ein Hystereseverhalten zeigen.

Anionische Polyaminosäure-Matrix:
Die erfindungsgemäß einzusetzenden anionischen Polyaminosäure-Matrices sind Polyglutaminsäure aufgebaut.. Das Molekulargewicht der erfindungsgemäß verwendeten anionischen Polyaminosäure-Matrices liegt im Bereich zwischen etwa 10 000 bis 2 000 000.
Anionisch modifizierte Polyaminosäuren sind aus der Natur bekannt und werden z. B. von verschiedenen Gram-positiven Bakterien produziert. Poly-gamma-Glutaminsäure wird u. a. von Bacillus licheniformis und Bacillus subtilis var. natto hergestellt.
Die biotechnologische Herstellung von anionischen Polyaminosäuren ist ebenfalls literatur-beschrieben. Die Arbeiten von M. Bovarnick et al. aus dem Jahre 1942 sind die frühesten Beispiele zur Herstellung von Polyglutaminsäure unter Einsatz von Mikroorganismen. Ferner ist die biotechnologische Herstellung von Polyglutaminsäure aus den Patentschriften JP 43-24 472 (1969) sowie der deutschen Offenlegungsschrift DE-A-198 25 507 (Bayer; 1999) bekannt.
Von den oben genannten anionischen Polyaminosäuren wird vorzugsweise die als Fermentationsprodukt erhältliche gamma-Polyglutaminsäure verwendet. Polyglutaminsäure ist ein hochviskoses, hochmolekulares Polymer der Glutaminsäure, welches freie Carboxylgruppen aufweist, welche dem Polymer einen insgesamt anionischen Charakter verleihen und die gute Wasserlöslichkeit verursachen. Polyglutaminsäure kann dabei auch in Form ihrer Alkalisalze, Ammoniumsalze, Ethanolaminsalze sowie Calciumsalze verwendet werden. Vorzugsweise wird aber die freie Säure selbst verwendet bzw. deren Partialneutralisate, welche mindestens noch 50 % freie Carboxylgruppen aufweisen.
Polyglutaminsäure ist als ein kosmetischer Feuchthaltefaktor zur topischen Applikation auf der Haut beschrieben, welcher sowohl direkt als auch indirekt den Wassergehalt in der Hornschicht der Haut positiv beeinflusst. Zum einen besitzt Polyglutaminsäure filmbildende Eigenschaften, zum anderen kann Polyglutaminsäure in Hautzellen die Produktion des hauteigenen Feuchthaltefaktors (NMF) erhöhen.
Der Einsatz von Polyglutaminsäure als Feuchthaltemittel für die Haut ist aus der JP-A-59-209 635 (Ajinomoto) bekannt. Es wird beschrieben, dass sie nach Applikation des jeweiligen Kosmetikums eine mäßige Feuchtigkeit und Glätte verleihen. Die zu erzielende Langzeitwirkung bei Verwendung üblicher, kommerziell erhältlicher Polyglutamintypen ist jedoch immer noch unzureichend.

Polysaccharide:
Polysaccharide stellen eine heterogene Gruppe unterschiedlich langer und unterschiedlich zusammengesetzter Polymere dar. Grundbausteine sind dabei jeweils Zuckerreste, die über glykosidische Bindungen untereinander verknüpft sind. Es gibt alpha-und beta-glykosidische Bindungen, die wiederum können zwischen einem C₁- (oder C₂-)Atom des einen Zuckerrests und dem C₂, C₃, C₄, C₅ oder C₆ des Zweiten liegen. Sind mehr als zwei Verknüpfungstypen in einem Molekül vereint, entstehen verzweigte Formen. Ein Polysaccharid kann aus einem Typ von Monomeren bestehen (Homopolymer), es können aber auch mehrere daran beteiligt sein (Heteropolymer). Der häufigste in Polysacchariden gefundene Zucker ist die Glucose, die durch sie gebildeten Polymere heißen Glucane.
Scleroglucan ist ein Polysaccharid, welches von bestimmten Pilzen wie z. B. *Sclerotium rolfsii* oder *Sclerotium glucanicum* sekretiert wird und als Hilfsstoff beim Abbau von Holz dient. Scleroglucan besteht aus β-1,3-Glucopyranose-Einheiten in der Hauptkette, wobei jede dritte Einheit mit einer zusätzlichen Glucoseeinheit β-1,6-D-glycosidisch verbunden ist. Im Gegensatz zu Beta-Glucanen aus anderen Quellen als Pilzen sind diese β-1,6-Verzweigungen nicht verlängert. In der nativen Form bildet Scleroglucan die für Beta-Glucane charakteristische Triple-Helix aus. Typische Molekulargewichte liegen im Bereich von 3 bis 5 x 10⁶, die maximale Löslichkeit in Wasser bei neutralem bis schwach saurem pH liegt bei ca. 1,5 bis 2 %. Es ist literaturbekannt, dass Scleroglucan wasserbindende und somit auch verdickende Eigenschaften aufweist. Daher findet Scleroglucan Anwendungen in der Kosmetik u. a. als Verdicker und Geliermittel. Solche Anwendungen sind beispielsweise beschrieben in der EP-A-0 979 642 (L'OREAL) bzw. im japanischen Patent JP-A-61-267 503 (SHISEIDO).
Scleroglucan ist kommerziell erhältlich und wird unter dem Markennamen ACTIGUM™ (Degussa) vertrieben. Neben der Funktion als Verdicker werden auch Moisturisation, Entzündungshemmung sowie ein angenehmes Hautgefühl als Eigenschaften ausgelobt.
Überaschenderweise zeigte niedermolekulares Skleroglucan ein höheres Wasserrückhaltevermögen und eine stärkere Verbesserung der Hautfeuchte verglichen mit hochmolekularem Skleroglucan.
Niedermolekulare Scleroglucane im Sinne der vorliegenden Erfindung bestehen im Wesentlichen aus nativen Scleroglucanen, welche durch saure, alkalische, oxidative und/oder enzymatische Hydrolyse gespalten werden. Gemäß der vorliegenden Erfindung werden Scleroglucane mit einem mittleren Molekulargewicht von weniger als 600 000g/mol, vorzugsweise von 100 000 bis 600 000 g/mol eingesetzt. Insbesondere bevorzugt sind Scleroglucane mit einem mittleren Molekulargewicht von 200 000 bis 500 000 g/mol und besonders bevorzugt 300 000 bis 400 000 g/mol. Damit weisen niedermolekulare Scleroglucane eine solch geringe Molekülgröße auf, dass diese Polysaccharide verbessert in der Lage sind in die Haut einzudringen. Weiterhin wirkt sich die geringe Molekülgröße auch vorteilhaft auf die Herstellung von interpenetrierenden Netzwerken mit anionischen Polyaminosäuren aus, da sich die Lösungseigenschaften und Kompatibilität mit der Polyaminosäure-Matrix verbessern, weil eine deutlich schwächere Verdickung auftritt, was wiederum höhere Einsatzkonzentrationen zulässt.

Interpenetrierende Polymernetzwerke:
Durch die Kombination der anionisch modifizierten Polyaminosäurematrix mit dem depolymerisierten Polysaccharid gemäß Anspruch 1 können in verschiedener Weise interpenetrierende Polymernetzwerke erhalten werden. Dabei werden im einfachsten Falle die Substanzen in einem geeigneten Verhältnis miteinander gemischt und in einem Lösungsmittel wie z. B. Wasser miteinander gemischt.
Die anionische Polyaminosäurematrix und das Polysaccharid können unvernetzt bleiben, müssen aber nach der erfindungsgemäßen Lehre ein homogenes, interpenetrierendes Polymernetzwerk ausbilden. Dabei können sich aber auch z. B. im Falle der Polyglutaminsäure (PGA) und dem depolymerisierten Scleroglucan die helixartigen Strukturen der Ausgangspolymere miteinander verschlaufen. Dabei wird das Polysaccharid in der Polyglutaminsäure-Matrix physikalisch eingefangen und beispielsweise über intermolekulare Wasserstoffbrücken ein quervernetztes interpenetrierendes Polymernetzwerk ausgebildet.
   Eine weitere Herstellmöglichkeit besteht darin die anionisch modifizierte Polyaminosäurematrix mit dem Polysaccharid zu vermischen, ggf. in Wasser aufzulösen und danach eine Vernetzung mittels ionisierender Strahlung zu erzielen. Alternativ können auch Verfahren durchgeführt werden, indem zunächst die anionisch modifizierte Polyaminosäurematrix durch Vernetzung mit ionisierender Strahlung in eine Hydrogelstruktur überführt wird und danach die Umsetzung mit dem depolymerisierten Polyaccharid stattfindet.
Ein weiteres Verfahren besteht darin eine chemische Vernetzung zwischen anionisch modifizierter Polyaminosäurematrix und depolymerisierten Polysaccharid durchzuführen, indem die Mischung beider Polymere mit einem reaktiven bifunktionellen Vernetzungsmittel behandelt werden, welches die Carbonsäuregruppen der anionisch modifizierten Polyaminosäurematrix mit den Hydroxyd-Grupppen des depolymerisierten Polysaccharides verknüpft. Solche Vernetzungsmittel können beispielsweise Di-Epoxide wie Ethylenglykoldiglycidylether sein.
   Methoden zur Bestimmung des Verschlaufungsgrades eines interpenetrierenden Polymernetzwerkes sind hinlänglich bekannt und beruhen auf den veränderten mechanischen und elektrischen Eigenschaften gegenüber einer reinen Mischung der Ausgangspolymeren. Die Quellkapazität, welche mit der Zahl der Vernetzungseinheiten im Polymernetzwerk korreliert, kann anhand üblicher Methoden bestimmt werden. Hierzu sei auf die Diskussion in der Fachliteratur unter "Molecular Interpenetrations of Sorption in Polymers Part 1", Advances in Polymer Science Vol. 99, Springer-Verlag, Berlin, Heidelberg, Deutschland (S. 22 bis 36, 1991) verwiesen. Unter Wasserzugabe bilden solche erfindungsgemäßen interpenetrierenden Polymernetzwerke Hydrogele auf, die sich durch eine ausgezeichnete Hautverträglichkeit und Hauthaftungsfähigkeit auszeichnen, ohne klebrig zu wirken. Diese interpenetrierenden Netzwerke können mit niedermolekularen Substanzen wie z. B. Osmoprotektantien beladen werden.

Anwendbarkeit:
Als erfindungsgemäße, kosmetische oder dermatologische Zubereitungen zur Regulierung der Hautfeuchtigkeit werden vor allem solche Mittel verstanden, die auf die Gesichtshaut und/oder andere Körperpartien aufgetragen werden.
Diese Mittel sind die üblicherweiser verwendeten kosmetischen oder dermatologischen Formulierungen, die im Allgemeinen als wässrige alkoholische Lösungen, Cremes, Emulsion, Lotionen, Gele, Aerosolspray oder -schaum, Nonaerosolspray oder -schaum vorliegen. Sie können demnach zur Anpassung auch weitere übliche Bestandteile enthalten, welche zur Behandlung, Pflege, Reinigung und dem Schutz der Haut dienen, wie beispielsweise hautkosmetischen Wirkstoffen (active ingredients) wie z. B. Ceramide, Pseudoceramide, Proteinhydrolysate pflanzlichen oder tierischen Ursprungs auf Basis Keratin, Collagen, Elastin, Weizen, Reis, Soja, Milch, Seide, Mais, Aminosäuren und Aminosäurederivate, entzündungshemmende Wirkstoffe, antimikriobelle Wirkstoffe, gebräuchliche Antioxidantien, Vitamine, Dexpanthenol, Milchsäure, Pyrrolidoncarbonsäure, Bisabolol sowie Pflanzen-, Hefe- und Algenextrakten.
Die Kombination mit üblicherweise verwendeten organischen oder anorganischen UV-Filtersubstanzen ist ebenfalls möglich und z. B. in Sonnenschutzpräparaten als besonders vorteilhaft anzusehen, da während der Anwendung das Austrocknen der Haut wirksam unterbunden werden kann und dadurch die natürliche Schutzfunktion der Haut erhalten bleibt. Zusätzlich können noch weitere kosmetische Hilfs-und Zusatzstoffe, die in solchen Zubereitungen üblich sind, enthalten sein. Solche Hilfstoffe sind z. B. Lösungsvermittler wie Ethanol, Isopropanol, Ehtylenglykol; Propylenglykol, Glycerin und Diethylenglykol. Zu den weiteren Komponenten zählen kosmetische Öle pflanzlichen und synthetischen Ursprungs, Emollients, Fette, Wachse, Rückfetter, Emulgatoren, Verdickungsmittel, anionische, zwitterionische, ampholytische und nichtionische Tenside sowie Duft- und Konservierungsstoffe.
Schließlich können die erfindungsgemäßen Formulierungen auch noch Komplexbildner wie EDTA, NTA, β-Alanindiessigsäure und Phosphonsäure, Farbstoffe zum Anfärben der kosmetischen Zubereitung, Trübungsmittel wie Latex, Styrol/PVP- und Styrol-Acrylamid-Copolymere, Perlglanzmittel wie Ethylenglykolmono-und -distearat und PEG-3-distearat, Pigmente, Lichtschutzmittel, Verdickungsmittel oder Treibmittel enthalten.
Typische Rahmenrezepturen für Hautbehandlungsmittel gehören zum bekannten Stand der Technik und sind beispielsweise in den Broschüren der Hersteller der jeweiligen Grund- und Wirkstoffe enthalten. Eine informative Quelle für derartige Formulierungen stellt z. B. das jährlich erscheinende Kosmetik-Jahrbuch (Herausgeber: B. Ziolkowsky, Verlag für Chemische Industrie) dar.
Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.
Die Herstellung der erfindungsgemäßen Formulierungen erfolgt in der üblichen Weise wobei die Moisturizer-Mischung bevorzugt in der wässrigen Phase der Formulierung gelöst wird oder nach der Herstellung der Formulierung bei Temperaturen unterhalb von 40 °C zugegeben wird. Die Einstellung des pH-Wertes erfolgt bevorzugt zuletzt durch Zugabe der dafür vorgesehenen Säure und/oder des Puffergemisches.

### Beispiel 1

### Herstellung einer Wirkstoffkombination

Die Herstellung der Wirkstoffkombination erfolgt folgendermaßen: das Wasser wird vorgelegt und alle wasserlöslichen Bestandteile, wie die Osmoprotektanten, mit Ausnahme der gamma-Polyglutaminsäure und des niedermolekularen depolymerisierten Skleroglucans, werden gelöst. Anschließend erwärmt man die Lösung auf ca. 60 - 70 °C und gibt unter starker Scherung (Ystrahl-Mischer, 10 - 20 000 min⁻¹) die Polyglutaminsäure und das Skleroglucan zu. Man rührt solange mit möglichst hoher Scherung, bis die Lösung homogen ist und kühlt anschließend unter leichtem Rühren auf 30 - 40°C ab.

### Wirkstoff-Kombination 1

| gamma-Polyglutaminsäure | |
|---|---|
| Anionische Polyaminosäure-Matrix, Komponente a) | 2,0% |
| Depolymerisiertes niedrigmolekulares | 2,0% |
| Skleroglucan(mittleres Molgewicht ca. 350 000) | |
| (Polysaccharid, Komponente b) | |
| Trimethylglycin (Komponente c) | 20,0% |
| Harnstoff | 10,0% |
| Kaliumlactat (Komponente c) | 5,0% |
| Wasser | 61,0% |

### Beispiel 2

### Herstellung einer Hautpflegeformulierung

Die Hautpflegeformulierung kann als wässrige Lotion, Wasser-in-Öl- oder Öl-in-Wasseremulsion, Öl- oder Öl-Alkohollotion, vesikulare Dispersion anionischer oder nichtionischer amphiphiler Lipide, wässrige, Wasser-Alkohol, Alkohol- oder Öl-Alkoholgel, fester Stick oder als Aerosol formuliert werden.

Wenn sie als Wasser-in-Öl oder Öl-in-Wasser-Emulsion formuliert wird, enthält der kosmetisch annehmbare Träger A vorzugsweise 5 bis 50 % einer Ölphase und 47 bis 94,95 % Wasser, bezogen auf den Gesamtanteil der Formulierung.

Die Ölphase kann jedes kosmetische Öl oder eine Mischung davon enthalten. Beispiele solcher Öle beinhalten aliphatische Kohlenwasserstoffe wie flüssiges Paraffin, Squalan, Vaseline und Ceresin, Pflanzenöle wie Olivenöl, Mandelöl, Sesamöl, Avocadoöl, Rizinusöl, Kakaobutter und Palmöl, Tieröle wie Haifischlebertran, Dorschleberöl, Walöl, Rindertalg und Butterfett, Wachse wie Bienenwachs, Karnaubapalmwachs, Spermazet und Lanolin, Fettsäuren wie Laurinsäure, Myristinsäure, Palmitinsäure, Stearinsäure, Ölsäure und Behensäure; aliphatische Alkohole wie Lauryl-, Stearyl-, Cetyl- und Oleylalkohol und aliphatische Ester wie Isopropyl-, Isocetyl- oder Octadecylmyristat, Butylstearat, Hexyllaurat, Diisopropylester der Adipinsäure oder Diisopropylsebacat. Bevorzugte Mono- oder Polyole für die Verwendung in Öl-Alkohol-Lotionen oder Öl-Alkohol- oder Alkoholgel, beinhalten Ethanol, Isopropanol, Propylenglykol, Hexylenglykol, Glycerin und Sorbit.

### O/W-Creme mit Wirkstoff-Kombination 1

| | | | |
|---|---|---|---|
| A) | TEGO® Care PS | Methyl Glucose sesquistearate | 3,0 % |
| | TEGOC® Alkanol 18 | Stearyl alcohol | 1,5 % |
| | TEGIN® M | Glyceryl Stearate | 3,5 % |
| | TEGOSOFT® liquid | Cetearyl Ethylhexanoate | 9,2 % |
| | TEGOSOFT® CT | Caprylic/capric triglyceride | 10,0 % |
| B) | Glycerin | | 3,0 % |
| | Wasser | | 62,65 % |
| C) | TEGO® Carbomer 134 | Carbomer | 0,2 % |
| | TEGOSOFT® liquid | Cetearyl-ethylhexanoate | 0,8 % |
| | Wirkstoffkombination 1 | | 5,0 % |
| | NaOH (10%) | | 0,65 % |
| | Parfüm | | 0,5 % |
| | Konservierungsmittel | | q. s. |

### Herstellung der Formulierung:

Phase A) und B) werden auf 65 °C erwärmt. Anschließend gibt man Phase B) zu A) und homogenisiert (z. B. mit einem Ultra Turrax). Die Emulsion wird unter leichtem Rühren abgekühlt bis auf 30 °C. Bei 60 °C wird Phase C) zugegeben und nochmal kurz homogenisiert. Die Wirkstoffkombination 1 sowie die Natronlauge und das Parfümöl werden nacheinander bei 35 - 40 °C zugegeben.

Für die nachfolgend beschriebenen Wirksamkeitsstudien wurde eine einfach aufgebaute O/W-Creme eingesetzt, die folgende Zusammensetzung aufwies:

**Tabelle 1**

| | |
|---|---|
| Ceteareth-25 | 2,0 % |
| Glyceryl Stearate | 4,0 % |
| Stearyl Alcohol | 2,0 % |
| Ethylhexyl Stearate | 8,5 % |
| Caprylic/Capric Triglyceride | 8,5 % |
| Konservierungsmittel | 0,1 % |
| Wasser | 64,9 % |
| Wirkstoffkombination 1 | 10,0 % |

### Beispiel 3

### Bestimmung der Moisturizer-Eigenschaften der Wirkstoffkombination

Die Corneometrie stellt eine nicht-invasive Methode dar, die auf Grund Veränderungen physikalischer Parameter im Stratum Corneum Rückschlüsse auf die Hautfeuchtigkeit gestattet. Zur praktischen Durchführung werden 12 bis 15 Probanden rekrutiert, deren Hautfeuchtigkeit vor und nach einmaliger Applikation der Testformulierungen mittels eines Corneometers HM99 (Courage & Khazaka, Köln, Deutschland) nach Angaben des Herstellers gemessen wird.

Nach einer Klimatisierung der Probanden über 15 Minuten unter definierten Bedingungen von 22 °C und 55 % relativer Feuchtigkeit wird die Hauthydratation corneometrisch bestimmt und im Anschluss daran 50 mg der Testformulierung auf einer Hautfläche mit einem jeweiligen Durchmesser von 2,5 cm aufgetragen und nach zwei Minuten etwaige Reste der Formulierung entfernt.

Nach einer zweistündigen Einwirkzeit wird erneut die Hauthydratation nach einer Klimatisierung von 15 Minuten (22 °C, 55 % rel. Feuchtigkeit) gemessen. Ein Anstieg der relativen Corneometereinheiten CU korreliert direkt mit einer verbesserten Hauthydratation.

Die Konzentrationen der Einzelsubstanzen wurden so gewählt, dass sie der Konzentration in 10% Wirkstoffkombination 1 entsprechen. Die folgende Tabelle (Tab. 2) zeigt die Zunahme der relativen Corneometereinheiten (CU) im Vergleich zur Leerformulierung.

**Tabelle 2**

| | ΔCU |
|---|---|
| 0,2% Skleroglucan | 1,1 |
| 0,2 % gamma-Polyglutamin-säure | 1,6 |
| 2,0% Trimethylglycin | 3,2 |
| 1,0% Harnstoff | 3,6 |
| 0,5 % Kaliumlactat | 1,6 |
| Wirstoffkombination 1 bestehend aus: | 14,2 |
| 0,2% Skleroglucan | |
| 0,2 % gamma-Polyglutamin-säure | |
| 2,0% Trimethylglycin | |
| 1,0% Harnstoff | |
| 0,5 % Kaliumlactat | |

Man erkennt, dass bereits die Einzelsubstanzen eine Verbesserung des Feuchtigkeitszustandes der Haut bewirken. Durch Kombination der verschiedenen Inhaltsstoffe wird diese Hydratation deutlich und synergistisch verstärkt. Während die Summe der Einzelkomponenten eine theroetische Verbesserung der Hautfeuchte um 11,1 Corneometereinheiten erwarten liesse, kommt es real zu einer Verbesserung von 14,2 Corneometereinheiten. Dies entspricht einer synergistischen Verbesserung durch die Kombination der Wirkstoffe von 27,9 %.

### Beispiel 4

### Bestimmung der in vitro-Sorption-Desorption-Eigenschaften

Um die in vitro-Sorption-Desorption-Eigenschaften von Moisturizern zu bestimmen, werden diese zuerst über Nacht bei 80 °C und 0 % relativer Feuchtigkeit (r.H.) getrocknet und anschließend im Exikator auf Raumtemperatur abgekühlt. Dann werden 4 - 5 g der Moisturizersubstanz in eine Petrischale gefüllt und 24 h bei 20 % r.H. gelagert. Die Gewichtszunahme wird protokolliert und die Luftfeuchtigkeit anschließend um 10 % erhöht. Nach 24 h wird das Gewicht des Moisturizers wieder kontrolliert. Dies wird schrittweise bis zu einer Luftfeuchtigkeit von 80 % wiederholt. Anschließend wird die Luftfeuchtigkeit wieder alle 24 h in 10 %-Schritten gesenkt und die Gewichtsabnahme protokolliert.

Von einem guten Moisturizer wird erwartet, dass er mit steigender Luftfeuchtigkeit möglichst große Mengen Wasser absorbiert. Dies ist z. B. bei Glycerin der Fall. Er sollte aber auch in der Lage sein, das absorbierte Wasser bei abnehmender Luftfeuchtigkeit zu speichern, wozu Glycerin nicht in der Lage ist. Betrachtet man dagegen z. B. die Sorption-Desorption-Kurve der Polyglutaminsäure, des Trimethylglycins oder auch des Harnstoffs, erkennt man neben einer sehr guten Wasseraufnahmefähigkeit auch, dass diese Substanzen in der Lage sind, das absorbierte Wasser bei abnehmender Luftfeuchtigkeit festzuhalten. Dieses Hysterese-Verhalten ist vorteilhaft für die Erhaltung der Hautfeuchtigkeit durch topische Applikation und sollte sich für einen Wirkstoff über einen möglichst grossen Feuchtigkeitsbereich erstrecken. Durch die Kombination der Inhaltsstoffe ist dies bei der Wirkstoffkombination 1 gegeben.

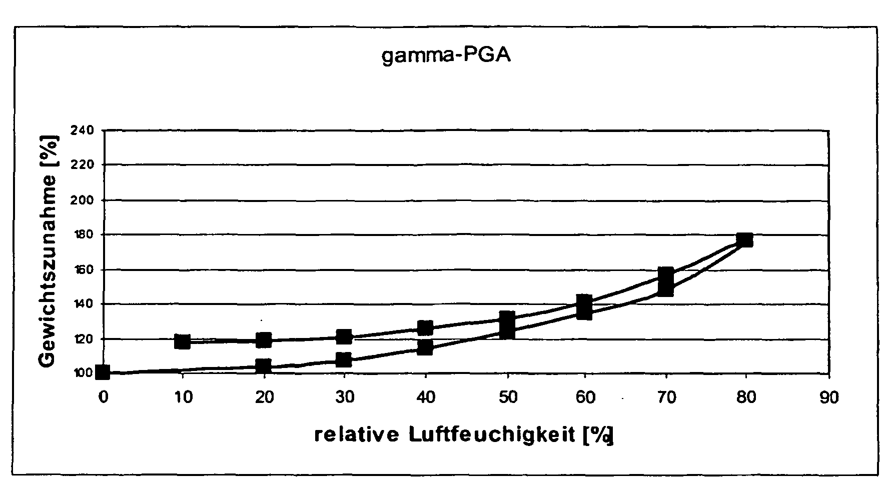

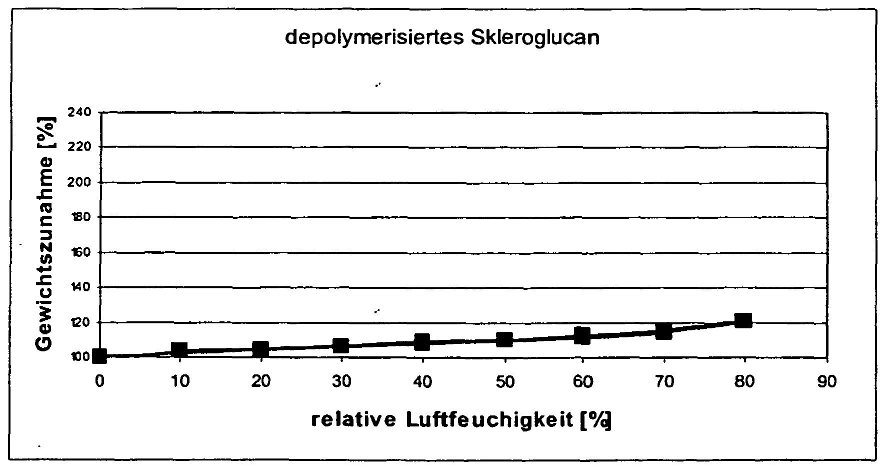

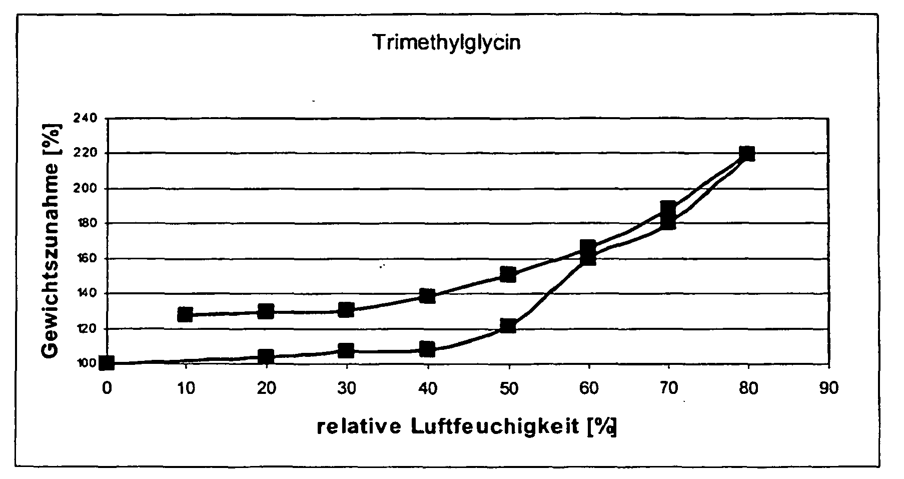

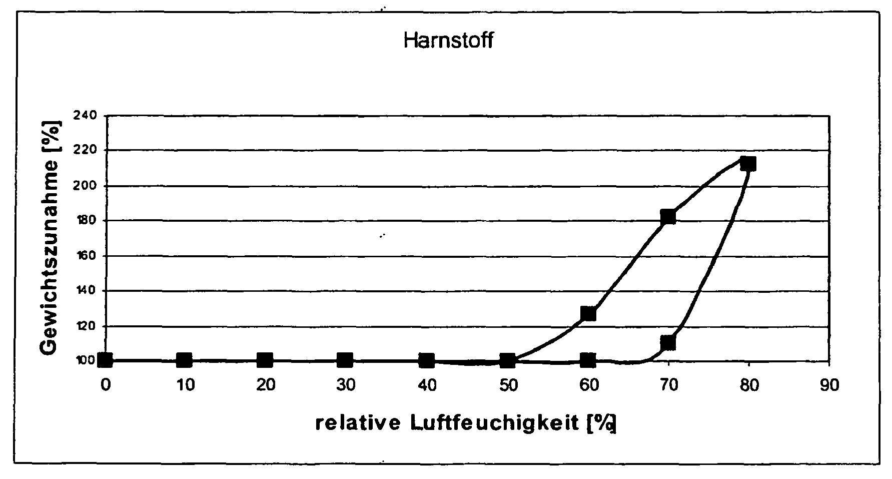

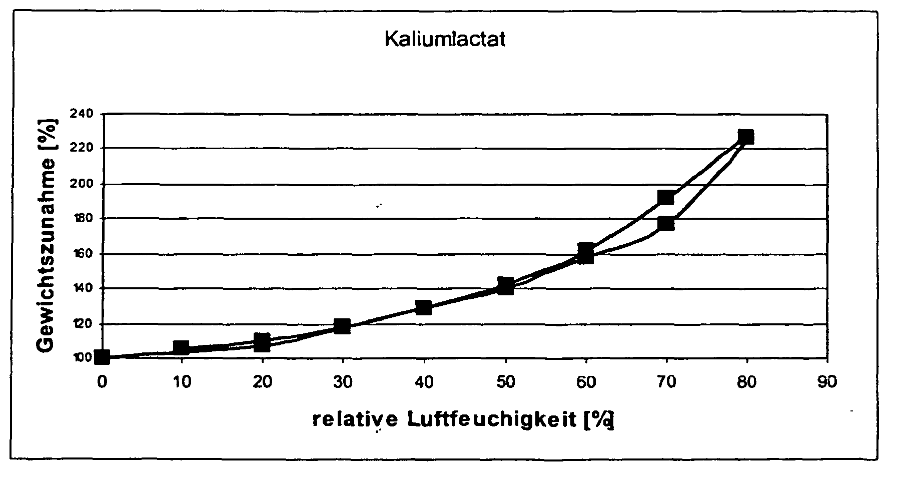

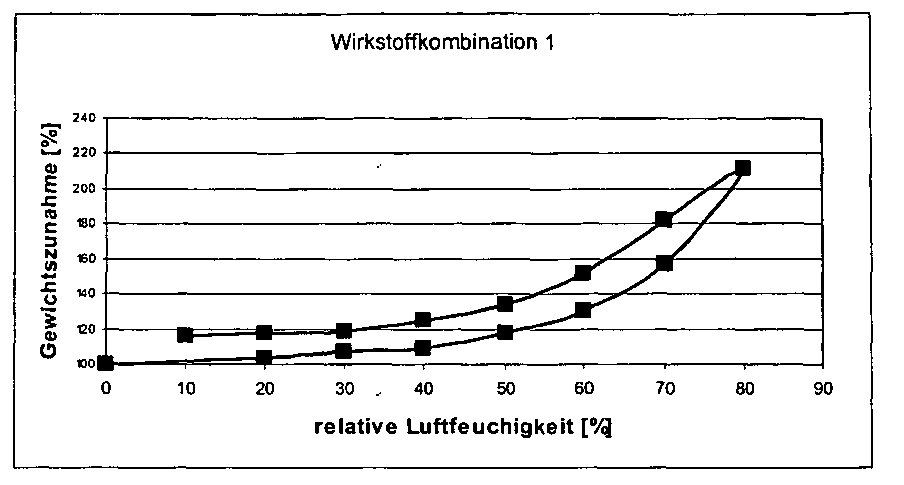

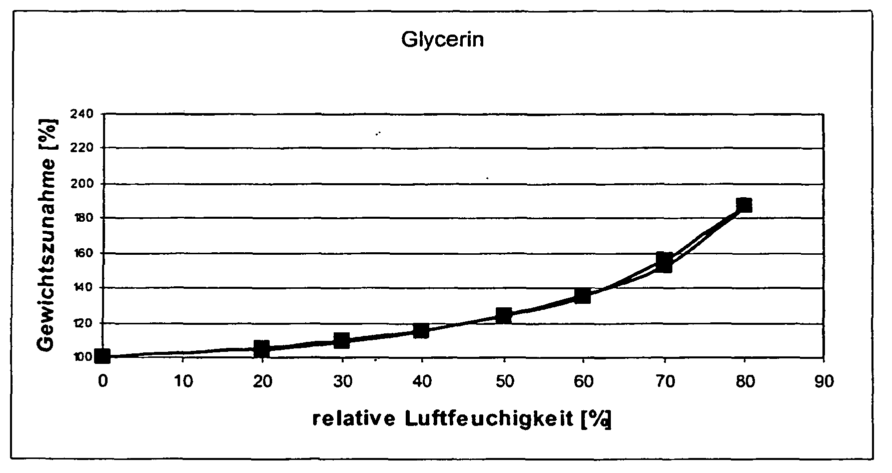

### Beispiel 5

### In vivo Sorption-Desorption

Zur Bestimmung der in vivo Sorption-Desorption-Eigenschaften der Wirkstoffkombination 1 wurde die O/W-Creme mit 10 % Wirkstoffkombination 1 (Zusammensetzung s. Tab. 1) von 3 Probanden 3 Wochen lang zweimal täglich auf die Unterarme aufgetragen. Nach 3 Wochen wurde zunächst mittels Corneometer HM99 der Hydratationszustand der Haut bestimmt. Anschließend wurde ein nasses Papiertuch auf den Unterarm gelegt (Einwirkzeit: 2 min), um die oberen Hautschichten mit Feuchtigkeit zu sättigen. Dann wurde die Hautoberfläche mit einem trockenen Papiertuch abgetupft und die Hautfeuchte gemessen. Die Hautfeuchte wurde anschließend in regelmäßigen Abständen kontrolliert.

Wie die folgende Abbildung zeigt, konnte die Haut, die mit der Wirkstoffkombination 1 behandelt worden war, zum einen mehr Wasser aufnehmen und zum anderen dieses Wasser auch stärker und länger zu binden. Dies Ergebnis zeigt die in vivo Wirkung der Ergebnisse aus Beispiel 4.

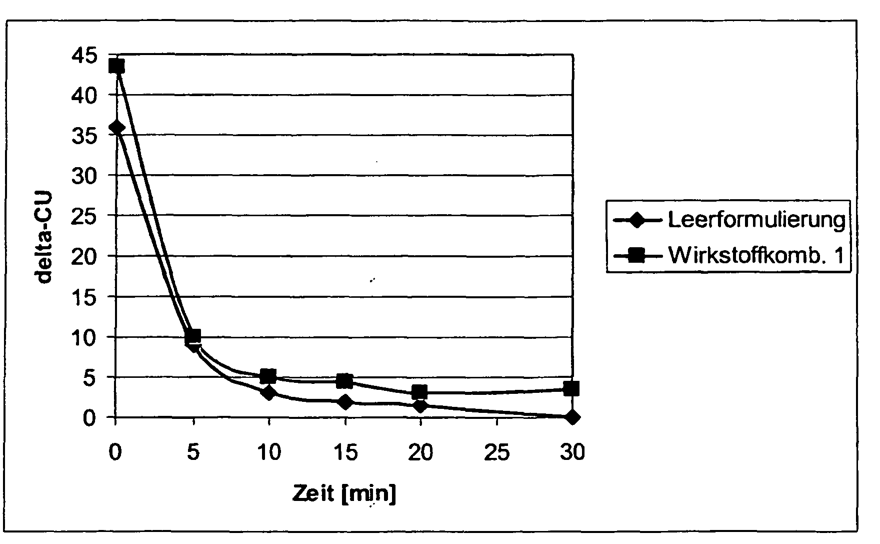

### Beispiel 6

### Langzeittest.

Die O/W-Creme (s. Tab. 1) mit 10 % Wirkstoffkombination sowie die entsprechende Leerformulierung wurden von 14 Probanden zweimal täglich über einen Zeitraum von 4 Wochen auf die Unterarme aufgetragen. Zu Beginn des Testes sowie in einwöchigem Abstand während der Behandlungsphase wurde die Hautfeuchtigkeit mit Hilfe des Corneometers HM99 kontrolliert. Nach 4 Wochen wurde die Behandlung abgebrochen und die Hautfeuchtigkeit nach 4 Tagen kontrolliert. Die folgende Abbildung zeigt die Zunahme der relativen Corneometereinheiten durch die Wirkstoffkombination 1 im Vergleich zur Leerformulierung.

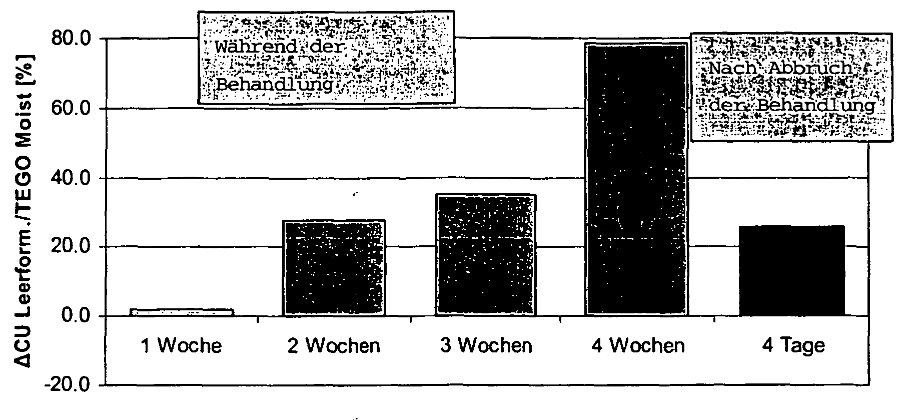

Mit zunehmender Behandlungdauer verbessert sich die Hydratation der Haut durch die Wirkstoffkombination 1 im Vergleich zur Leerformulierung signifikant. Diese Verbesserung ist auch noch 4 Tage nach Abbruch der Behandlung nachweisbar.

Während der 4wöchigen Anwendung wurden außerdem die Hautrauhigkeit und das Hautbild bewertet. Dies geschah mit Hilfe von D-Squame-Tapes (durchsichtige Klebe-Tapes), die mit definiertem Druck auf den Unterarm aufgeklebt und anschließend abgezogen wurden, um so die obersten, losen Hautschuppen zu entfernen. Die Menge an Hautschuppen wurde quantitativ ermittelt, indem die D-Squame-Tapes mit NaOH versetzt wurden, um die Proteine zu lösen. Anschließend wurde mit HCl neutralisiert und die Konzentration der gelösten Proteine mittels Bradford-Test bestimmt.

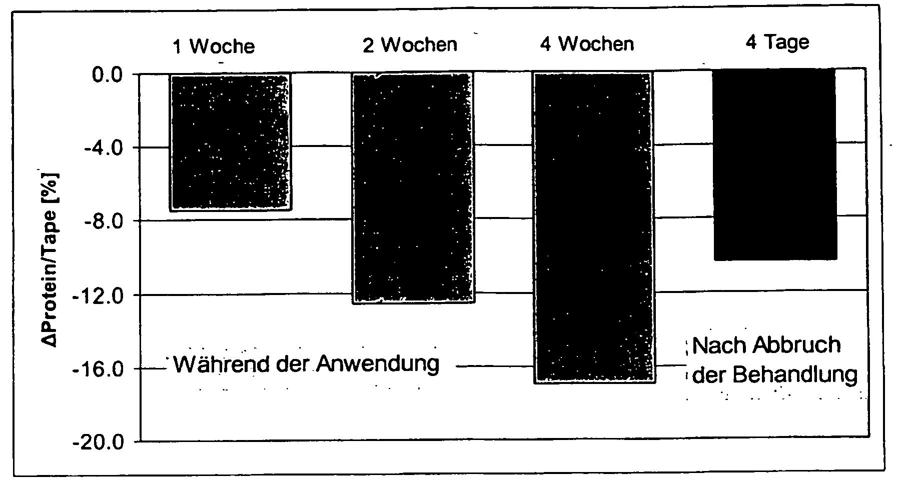

Die Grafik zeigt die Differenz der Protein-Konzentration zwischen der Haut, die mit Wirkstoffkombination 1 behandelt wurde und der mit der Leerformulierung behandelten Haut. Je geringer die Protein-Konzentration ist, desto weniger rau ist die Hautoberfläche.

Mit zunehmender Behandlungsdauer wird die Hautrauigkeit durch Behandlung mit der Wirkstoffkombination 1 im Vergleich zu Leerformulierung deutlich geringer. Die Hautrauigkeit verändert sich auch nach Abbruch der Behandlung nur wenig, d. h. der pflegende Effekt der Wirkstoffkombination 1 hält weiter an.

Dies konnte auch durch die visuelle Beurteilung der Größe der Hautschuppen auf den D-Squame Tapes bestätigt werden. Dazu wurden die Tapes anhand einer Skala von 1 - 5 (1 = sehr feine Schuppen, 5 = sehr große Schuppen) visuell beurteilt. Die folgende Grafik zeigt die Verbesserung der mit Wirkstoffkombination 1 behandelten Haut im Vergleich zur Haut, die mit der Leerformulierung behandelt wurde:

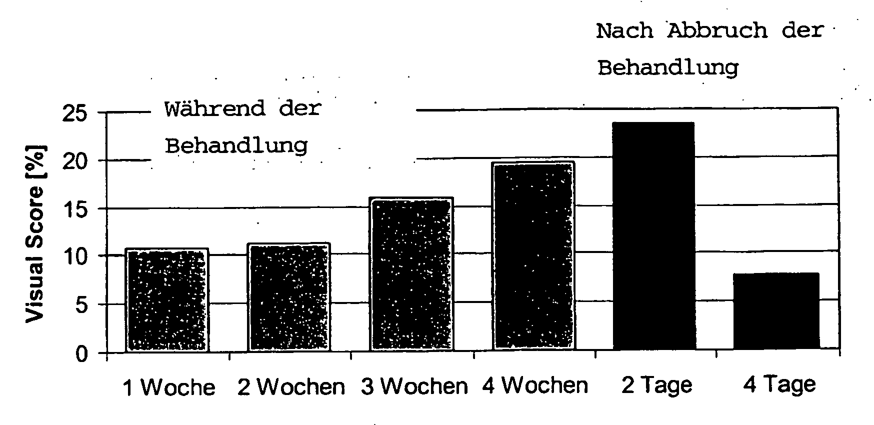

Die Hautstruktur verbessert sich signifikant während des Behandlungszeitraums. Dieser Effekt bleibt auch nach Abbruch der Behandlung erhalten.

### Beispiel 7

### Einfluss auf die Hautelastizität

Zur Bestimmung des positiven Einflusses der Wirkstoffkombination 1 auf die Hautelastizität wurde die in Tabelle 3 beschriebene O/W-Creme mit 5 % Wirkstoffkombination von 12 Probanden 4 Wochen lang zweimal täglich auf die Innenseite der Unterarme aufgetragen. Zu Beginn des Testes sowie nach 4 Wochen wurde die Hautelastizität mit einem Cutometer MPA 580 von Courage & Khazaka kontrolliert.

Bei der Bestimmung der Hautelastizität mit einem Cutometer wird die Haut durch Unterdruck (450 mbar) in eine Sondenöffnung gesogen. Der Durchmesser dieser Sondenöffnung beträgt 2 mm. Die Dehnung der Haut wird 5s aufrecht gehalten, anschließend wird die Haut durch Aufheben des Unterdrucks wieder entspannt. Dieser Zyklus aus Dehnen und Entspannen wird dreimal wiederholt. Aus der erhaltenen Kurve lassen sich u.a. die folgenden Elastizitätsparameter berechnen:
- Der Parameter R1 stellt die Höhe der Restauslenkung nach dem ersten Messzyklus dar. Er beschreibt also die Rückbildungsfähigkeit der Haut.
- R2 ist die sogenannte Bruttoelastizität, der Quotient aus UA und UF, wobei UF die maximale Ausdehnung der Haut am Ende des ersten Messzyklus darstellt und UA die Differenz aus UF und R1 ist.
- R5 ist die sogenannte Nettoelastizität, der Quotient aus UR und UE, wobei UR die Höhe der elastischen Dehnung beim Entspannen, UE die Höhe der elastischen Dehnung der Haut beim Einsaugen in die Sonde darstellt.
- R7 ist der Quotient aus UR und UF, d.h. es stellt das Verhältnis des elastischen Anteils nach Abschalten des Vakuums zur Gesamtauslenkung des ersten Zyklus dar.
- F0 ist die Fläche innerhalb des Rechtecks, gebildet aus UF x Ansaugzeit, oberhalb der Kurve. Ein vollständig elastisches Material würde keine Fläche zeigen.
Weitere Informationen zur Bestimmung der Hautelastizität mittels Cutometer können der Bedienungsanleitung des Cutometers entnommen werden (Information und Gebrauchsanweisung zum Cutometer MPA 580, CK electronic GmbH, Köln).

**Tabelle 3**

| O/W Creme | |
|---|---|
| Ceteareth-25 | 2.0 % |
| Bis-PEG/PPG-16/16 PEG/PPG Dimethicone; | 1.0 % |
| Caprylic/Capric Triglyceride | |
| Stearic Acid | 0.5 % |
| Cetearyl Alcohol | 5.0 % |
| C12-15 Alkyl Benzoate | 9.0 % |
| PPG-3 Myristyl Ether | 9.0 % |
| Wasser | 67.32 % |
| NaOH (10%) | 0.43 % |
| Carbomer | 0.15 % |
| Ethylhexyl Palmitate | 0.60 % |
| Wirkstoffkombination 1 | 5.0 % |

Die folgende Tabelle gibt die Verbesserung der verschiedenen Elastizitätsparameter nach 4-wöchiger Anwendung der Testformulierung wieder.

**Tabelle 4: Verbesserung der Elastizitätsparameter nach vierwöchiger Anwendung der Testformulierungen.**

| Elastizitätsparameter | Vehikel | 5% Wirkstoffkombination 1 |
|---|---|---|
| R1 | 0,0 % | 21,8 % |
| R2 | -0,4 % | 1,6 % |
| R5 | -1,5 % | 2,3 % |
| R7 | -2,4 % | 0,7 % |
| F0 | 4,0 % | 9,9 % |

Bei den Parametern R1 und F0 zeigt sich eine deutliche Verbesserung der Hautelastizität durch die Wirkstoffkombination 1. Betrachtet man die Parameter R2, R5 und R7, bewirkt das Vehikel eine schwache Verschlechterung, während die Wirkstoffkombination einen leicht positiven Einfluss auf die Hautelastizität hat.

## Patentansprüche

1. Kosmetische und oder dermatologische Formulierungen zur lokalen topischen Behandlung von Körperteilen zur Regulierung und Verbesserung des Feuchtigkeitsgehalts der Haut, enthaltend als Wirkstoffkombination
a) mindestens eine anionische Polyaminosäure-Matrix
b) mindestens ein Polysaccharid und gegebenenfalls
c) ein oder mehrere Osmoprotectanten
für die topische Anwendung, **dadurch gekennzeichnet, dass** die anionische Polyaminosäure eine Polyglutaminsäure mit einem mittleren Molgewicht von 1 000 000 bis 2 000 000 g/mol ist, und dass das Polysaccharid Scleroglucan mit einem mittleren Molgewicht von weniger als 600 000 g/mol ist.

2. Kosmetische und oder dermatologische Formulierungen gemäß Anspruch 1, **dadurch gekennzeichnet, dass** das Polysaccharid Scleroglucan mit einem mittleren Molgewicht von weniger als 500 000 g/mol ist.

3. Kosmetische und oder dermatologische Formulierungen gemäß Anspruch 1, **dadurch gekennzeichnet, dass** das Polysaccharid Scleroglucan mit einem mittleren Molgewicht von weniger als 400 000 g/mol ist.

4. Kosmetische und oder dermatologische Formulierungen gemäß mindestens einem der Ansprüche 1 bis 3, **dadurch gekennzeichnet, dass** die Wirkstoffkombination aus
a) mindestens einer anionischen Polyaminosäure-Matrix und
b) mindestens einer Polysaccharid-Matrix in Form eines interpenetrierenden Polymernetzwerkes vorliegt.

5. Kosmetische Zusammensetzungen gemäß mindestens einem der Ansprüche 1 bis 4, bestehend aus einer Wirkstoffkombination aus mindestens einer Polysaccharid-Matrix und mindestens einer anionischen Polyaminosäure-Matrix, welche ein Hysterese-Verhalten bezüglich der Wasserbindung aufweist.

6. Kosmetische und oder dermatologische Formulierungen gemäß mindestens einem der Ansprüche 1 bis 5, **dadurch gekennzeichnet, dass** das Verhältnis der Komponenten a) : b) im Bereich von 1/99 bis 99/1 liegt.

7. Kosmetische und oder dermatologische Formulierungen gemäß mindestens einem der Ansprüche 1 bis 6, **dadurch gekennzeichnet, dass** als Osmoprotectant c) mindestens eine Verbindung verwendet wird ausgesucht aus der Gruppe Trimethylglycin, Arginin, Kreatin, Kaliumlactat, Taurin, Trehalose, Myoinositol.

8. Kosmetische Zusammensetzungen gemäß mindestens einem der Ansprüche 1 bis 7 **dadurch gekennzeichnet, dass** sie
a) eine Polyglutaminsäure in einer Menge zwischen 0,05 und 5 % und
b) mindestens ein depolymerisiertes Scleroglucan in einer Menge zwischen 0,05 und 5 % und
c) mindestens ein oder mehrere Osmoprotectanten in einer Menge zwischen 0,01 und 25 %
enthalten.

9. Kosmetische und oder dermatologische Formulierungen gemäß mindestens einem der Ansprüche 1 bis 8, **dadurch gekennzeichnet, dass** als weitere Wirkstoffe mindestens eine Verbindung verwendet wird ausgesucht aus der Gruppe der Vitamine, Peptide, Ceramide, Sphingolipide und/oder Antioxidantien.

10. Kosmetische Zusammensetzungen gemäß mindestens einem der Ansprüche 1 bis 9, **dadurch gekennzeichnet, dass** sie mindestens eine weitere Komponente ausgesucht aus der Gruppe Harnstoff, Glycerin, Niacinamid, Panthenol, Allantoin, Bisabolol, Pyrrolidoncarbonsäure, Proteine, Proteinhydrolysate, Aminosäuren, Propylenglykol, Phospholipide, Lysophospholipide sowie Pflanzen-, Bakterien oder Pilzextrakte enthalten.

## Claims

1. Cosmetic and/or dermatological formulations for the local topical treatment of parts of the body for regulating and improving the moisture content of the skin, comprising as active ingredient combination
a) at least one anionic polyamino acid matrix
b) at least one polysaccharide and optionally
c) one or more osmoprotectants
for topical application, **characterized in that** the anionic polyamino acid is a polyglutamic acid with an average molecular weight of from 1 000 000 to 2 000 000 g/mol, and **in that** the polysaccharide is scleroglucan with an average molecular weight of less than 600 000 g/mol.

2. Cosmetic and/or dermatological formulations according to Claim 1, **characterized in that** the polysaccharide is scleroglucan with an average molecular weight of less than 500 000 g/mol.

3. Cosmetic and/or dermatological formulations according to Claim 1, **characterized in that** the polysaccharide is scleroglucan with an average molecular weight of less than 400 000 g/mol.

4. Cosmetic and/or dermatological formulations according to at least one of Claims 1 to 3, **characterized in that** the active ingredient combination of
a) at least one anionic polyamino acid matrix and
b) at least one polysaccharide matrix is present in the form of an interpenetrating polymer network.

5. Cosmetic composition according to at least one of Claims 1 to 4, consisting of an active ingredient combination of at least one polysaccharide matrix and at least one anionic polyamino acid matrix which has hysteresis behavior with regard to water binding.

6. Cosmetic and/or dermatological formulations according to at least one of Claims 1 to 5, **characterized in that** the ratio of components a) : b) is in the range from 1/99 to 99/1.

7. Cosmetic and/or dermatological formulations according to at least one of Claims 1 to 6, **characterized in that** the osmoprotectant c) used is at least one compound chosen from the group consisting of trimethylglycine, arginine, creatine, potassium lactate, taurine, trehalose, myoinositol.

8. Cosmetic compositions according to at least one of Claims 1 to 7, **characterized in that** they comprise
a) a polyglutamic acid in an amount between 0.05 and 5% and
b) at least one depolymerized scleroglucan in an amount between 0.05 and 5% and
c) at least one or more osmoprotectants in an amount between 0.01 and 25%.

9. Cosmetic and/or dermatological formulations according to at least one of Claims 1 to 8, **characterized in that** the further active ingredients used are at least one compound chosen from the group of vitamins, peptides, ceramides, sphingolipids and/or antioxidants.

10. Cosmetic compositions according to at least one of Claims 1 to 9, **characterized in that** they comprise at least one further component chosen from the group consisting of urea, glycerol, niacinamide, panthenol, allantoin, bisabolol, pyrrolidonecarboxylic acid, proteins, protein hydrolysates, amino acids, propylene glycol, phospholipids, lysophospholipids, and plant extracts, bacterial extracts or fungal extracts.

## Revendications

1. Compositions cosmétiques et/ou dermatologiques destinées au traitement topique local de parties du corps pour la régulation et l'amélioration de la teneur en humidité de la peau, contenant en tant qu'association de substances actives
a) au moins une matrice poly(aminoacide) anionique
b) au moins un polysaccharide et éventuellement
c) un ou plusieurs osmoprotecteurs
pour l'application topique, **caractérisées en ce que** le poly(aminoacide) anionique est un poly(acide glutamique) ayant une masse moléculaire moyenne de 1 000 000 à 2 000 000 g/mole, et **en ce que** le polysaccharide est un scléroglucane ayant une masse moléculaire moyenne de moins de 600 000 g/mole.

2. Compositions cosmétiques et/ou dermatologiques selon la revendication 1, **caractérisées en ce que** le polysaccharide est un scléroglucane ayant une masse moléculaire moyenne de moins de 500 000 g/mole.

3. Compositions cosmétiques et/ou dermatologiques selon la revendication 1, **caractérisées en ce que** le polysaccharide est un scléroglucane ayant une masse moléculaire moyenne de moins de 400 000 g/mole.

4. Compositions cosmétiques et/ou dermatologiques selon au moins l'une des revendications 1 à 3, **caractérisées en ce que** l'association de substances actives consiste en
a) au moins une matrice poly(aminoacide) anionique et
b) au moins une matrice polysaccharidique sous forme d'un réseau polymère interpénétrant.

5. Compositions cosmétiques selon au moins l'une des revendications 1 à 4, consistant en une association de substances actives constituée d'au moins une matrice polysaccharidique et d'au moins une matrice poly(aminoacide) anionique qui présente un comportement d'hystérésis eu égard à la fixation de l'eau.

6. Compositions cosmétiques et/ou dermatologiques selon au moins l'une des revendications 1 à 5, **caractérisées en ce que** le rapport des composants a):b) se situe dans la plage allant de 1/99 à 99/1.

7. Compositions cosmétiques et/ou dermatologiques selon au moins l'une des revendications 1 à 6, **caractérisées en ce qu'**on utilise comme osmoprotecteur c) au moins un composé choisi dans l'ensemble constitué par la triméthylglycine, l'arginine, la créatine, le lactate de potassium, la taurine, le tréhalose, le myo-inositol.

8. Compositions cosmétiques selon au moins l'une des revendications 1 à 7, **caractérisées en ce qu'**elles contiennent
a) un poly(acide glutamique) en une quantité comprise entre 0,05 et 5 % et
b) au moins un scléroglucane dépolymérisé en une quantité comprise entre 0,05 et 5 % et
c) au moins un ou plusieurs osmoprotecteurs en une quantité comprise entre 0,01 et 25 %.

9. Compositions cosmétiques et/ou dermatologiques selon au moins l'une des revendications 1 à 8, **caractérisées en ce qu'**on utilise comme autres substances actives au moins un composé choisi dans l'ensemble constitué par des vitamines, des peptides, des céramides, des sphingolipides et/ou des antioxydants.

10. Compositions cosmétiques selon au moins l'une des revendications 1 à 9, **caractérisées en ce qu'**elles contiennent au moins un autres composant choisi dans l'ensemble constitué par l'urée, le glycérol, la niacinamide, le panthénol, l'allantoïne, le bisabolol, l'acide pyrrolidonecarboxylique, des protéines, des hydrolysats de protéine, des aminoacides, le propylèneglycol, des phospholipides, des lysophospholipides ainsi que des extraits de plantes, de bactéries ou de champignons.
